# EUROPEAN PATENT APPLICATION

(11) **EP 2 979 646 A1**
(43) Date of publication of application: **03.02.2016**
(21) Application number: 15179107.6
(22) Date of filing: 30.07.2015
(51) Int. Cl.: A61B 17/00, A61B 17/221

(54) **INTRALUMINAL DEVICE FOR TREATING VARICOSE VEINS**

(30) Priority: 31.07.2014 US 201414447849
(71) Applicant: Terumo Kabushiki Kaisha, Tokyo 151-0072 (JP)
(72) Inventor: HAYAKAWA, Kouichi, Nakai-machi, Kanagawa 259-0151 (JP); AKIMOTO, Yuri, Nakai-machi, Kanagawa 259-0151 (JP)
(74) Representative: Casalonga

(57) **Abstract**

A method of treating a varicose vein involves inserting the distal portion of an intraluminal device into a varicose vein, wherein the device includes an elongated outer sheath and an expandable expansion member at the distal end of the outer sheath. The method also involves relatively moving the distal and proximal ends of the expandable spiral expansion member to reduce the distance between the distal and proximal ends of the expansion member and cause the expansion member positioned inside the varicose vein to outwardly expand to an expanded expansion member comprising a plurality of contact members in contact with the inner wall of the varicose vein. The method further includes axially moving the expanded expansion member while the contact members are in contact with the inner wall of the varicose vein and rotating the expanded expansion member during the axial movement.

## Description

### TECHNICAL FIELD

The invention pertains to a method of treating veins and an intraluminal device used in performing such method. More specifically, the invention involves a method of treating varicose veins and an intraluminal device for such method

### BACKGROUND DISCUSSION

Veins in the human body possess leaflet valves that prevent blood from flowing backwards (reflux). Leg muscles typically pump the veins to return blood to the heart against the effects of gravity. When veins become varicose (i.e., varicose veins), the leaflets forming the leaflet valves no longer properly meet to close, and so the valves do not properly work. This allows the backward flow of blood into the veins, causing the veins to enlarge. Varicose veins are thus veins that have become enlarged and tortuous. Varicose veins are somewhat more common in the superficial veins of the legs, which are typically subject to relatively high pressure when standing.

Devices have been proposed in the past to treat varicose veins by occluding the blood vessel (varicose vein). These devices typically operate to occlude the blood vessel in one of two ways. One way involves thermal ablation, in which anesthesia is extravascularly injected to decrease the diameter of the vessel, and then applying energy to the vessel. The other method is a non-thermal ablation that involves stimulating the inner wall of the blood vessel intravascularly to decrease the diameter of the blood vessel, and then injecting sclerosant to occlude the blood vessel.

The treatment of varicose veins through use of ablation devices typically involves the use of tumescent local anesthesia (TLA) that creates spasms in the blood vessel or vein. However, TLA is performed percutaneously and is thus a rather invasive procedure. In addition, the tumescent local anesthesia is typically injected into many places, and thus requires a significant amount of treatment time.

Various publications describe known method and apparatus for treating varicose veins through use of energy or physical stimulation. Examples include U.S. Patent No. 7,396,355 describing the use of energy in combination with tumescent tissue compression, U.S. Patent No. 7,077,836 describing an apparatus and method for delivering a sclerosing agent to a varicose vein, U.S. Patent No. 7,862,575 disclosing a vascular ablation apparatus that delivers a sclerosant to a varicose vein while the vein is being disrupted or irritated, and U.S. Patent No. 6,402,745, describing a method and device for collapsing varicose veins through use of an intravenous surgical instrument having an electrode at its tip.

### SUMMARY

According to one aspect, a method of treating a vein comprises inserting a portion of an intraluminal device into a vein, wherein the intraluminal device comprises an elongated inner member positioned inside an elongated outer sheath, the elongated inner member possessing a distal end portion and the elongated outer sheath possessing a distal end portion and a proximal end portion, the intraluminal device also including an expandable spiral expansion member positioned between the distal end portion of the elongated inner member and the distal end portion of the elongated outer sheath, the elongated inner member being fixed to a distal end portion of the expandable spiral expansion member and the distal end portion of the elongated outer sheath being fixed to a proximal end of the expandable spiral expansion member so that movement of the elongated inner member relative to the elongated outer sheath axially expands and contracts the expandable spiral expansion member. The method also involves moving the elongated inner member in a proximal direction relative to the elongated outer sheath while the expandable spiral expansion member is positioned in the vein to cause the expandable spiral expansion member to move in the proximal direction relative to the proximal end portion of the elongated outer sheath, with the movement of the expandable spiral expansion member relative to the proximal end portion of the elongated outer sheath causing the expandable spiral expansion member positioned inside the vein to outwardly expand to an expanded state so that the spiral expansion member becomes an expanded spiral expansion member, and the expanded spiral expansion member comprising a plurality of spirally extending spiral members in contact with the inner wall of the vein. The method further includes axially moving the expanded spiral expansion member while the spiral members are in contact with the inner wall of the vein and rotating the expanded spiral expansion member during the axial movement so that the expanded spiral expansion member which is in contact with the inner wall of the vein damages the inner wall of the vein, injecting fluid into the vein, and withdrawing the intraluminal device from the vein.

According to another aspect, a method of treating a vein comprises: inserting a distal portion of an intraluminal device into a vein, with the intraluminal device comprising an elongated outer sheath possessing a distal end, an expandable expansion member at the distal end of the outer sheath, the expandable expansion member possessing a distal end and a proximal end; relatively moving the distal and proximal ends of the expandable spiral expansion member to reduce the distance between the distal and proximal ends of the expansion member and cause the expansion member positioned inside the vein to outwardly expand to an expanded expansion member comprising a plurality of contact members in contact with the inner wall of the vein; axially moving the expanded expansion member while the contact members are in contact with the inner wall of the vein and rotating the expanded expansion member during the axial movement so that the contact members of the expanded expansion member which are in contact with the inner wall of the vein damage the inner wall of the vein; injecting fluid into the vein; and withdrawing the intraluminal device from the vein.

Another aspect of the disclosure here involves a device configured to be positioned in a vein to treat the vein. The device comprises: an outer sheath possessing a proximal end portion and a distal end portion; an elongated inner member positioned inside the elongated outer sheath and possessing a distal end portion, and an expandable spiral expansion member possessing a proximal end portion and a distal end portion, with the spiral expansion member being positioned between the distal end of the elongated outer sheath and the distal end portion of the elongated inner member, the elongated inner member being fixed to the distal end portion of the expandable spiral expansion member, and the distal end portion of the elongated outer sheath being fixed to the expandable spiral expansion member. The elongated inner member is axially movable relative to the elongated outer sheath when the device is positioned in the vein so that axial movement of the elongated inner member in a proximal direction relative to the elongated outer sheath axially moves the distal end of the expandable spiral expansion member toward and relative to the proximal end of the expandable spiral expansion member such that the expandable spiral expansion member outwardly expands to an expanded state so that the spiral expansion member becomes an expanded spiral expansion member comprising a plurality of spirally extending contact members in contact with the inner wall of the vein. The expanded spiral expansion member is axially movable along, and rotatable relative to, the vein while the spirally extending contact members are in contact with the inner wall of the vein to damage the inner wall of the vein.

### BRIEF DESCRIPTION OF THE DRAWINGS

Additional details, characteristics and aspects of the method and device disclosed here will become more apparent from the following detailed description considered with reference to the accompanying drawing figures. It is to be understood that the drawings are not intended to accurately depict the relative dimensions of features associated with the intraluminal device and/or the blood vessel (varicose vein) into which the intraluminal device is inserted.
FIG. 1 is a somewhat schematic side view of an intraluminal device (assembly) representing an example of the intraluminal device disclosed here.
FIG. 1A is a somewhat schematic side view of a modified form of the intraluminal device (assembly) shown in FIG. 1 representing a modified version of the intraluminal device.
FIG. 2 is a somewhat schematic side view of the intraluminal device shown in FIG. 1, depicting the expansion member in the expanded state.
FIGS. 3A-3E illustrate sequentially performed aspects of the manner of operation and use of the intraluminal device shown in FIGS. 1 and 2 representing an example of the manner of operation and use disclosed here.
FIG. 4 is a somewhat schematic side view of an embodiment of the intraluminal device (assembly) representing another example of the intraluminal device disclosed here.
FIG. 5 is a somewhat schematic side view of the intraluminal device shown in FIG. 4, depicting the expansion member in the expanded state.
FIGS. 6A-6D illustrate sequentially performed aspects of the manner of operation and use of the intraluminal device shown in FIGS. 4 and 5 representing another example of the manner of operation and use disclosed here.
FIG. 7 is a somewhat schematic side view of an embodiment of the intraluminal device (assembly) representing another example of the intraluminal device disclosed here.
FIG. 8 is a somewhat schematic side view of the intraluminal device shown in FIG. 7, depicting the expansion member in the expanded state.
FIGS. 9A-9D illustrate sequentially performed aspects of the manner of operation and use of the intraluminal device shown in FIGS. 7 and 8 representing another example of the manner of operation and use disclosed here.
FIG. 10 is a somewhat schematic side view of an embodiment of the intraluminal device (assembly) representing another example of the intraluminal device disclosed here.
FIG. 11 is a somewhat schematic side view of the intraluminal device shown in FIG. 10, depicting the expansion member in the expanded state.
FIGS. 12A-12D illustrate sequentially performed aspects of the manner of operation and use of the intraluminal device shown in FIGS. 10 and 11 representing another example of the manner of operation and use disclosed here.
FIG. 13 is a somewhat schematic side view of an embodiment of the intraluminal device (assembly) representing another example of the intraluminal device disclosed here.
FIG. 14 is a somewhat schematic side view of the intraluminal device shown in FIG. 13, depicting the expansion member in the expanded state.
FIGS. 15A-15E illustrate sequentially performed aspects of the manner of operation and use of the intraluminal device shown in FIGS. 13 and 14 representing another example of the manner of operation and use disclosed here.
FIG. 16 is a somewhat schematic side view of an embodiment of the intraluminal device (assembly) representing another example of the intraluminal device disclosed here.
FIG. 17 is a somewhat schematic side view of the intraluminal device shown in FIG. 16, depicting the expansion member in the expanded state.
FIGS. 18A-18E illustrate sequentially performed aspects of the manner of operation and use of the intraluminal device shown in FIGS. 16 and 17 representing another example of the manner of operation and use disclosed here.
FIG. 19A is a distal end view of the further expanded expansion member expanded in the second expanded state and FIG. 19B is a side view of the further expanded expansion member expanded in the second expanded state.
FIG. 20 is an enlarged side view of the expanded expansion member which can be used with any of the embodiments of the intraluminal device disclosed and illustrated here.
FIG. 21 is a somewhat schematic side view of an embodiment of the intraluminal device (assembly) representing another example of the intraluminal device disclosed here.
FIG. 22 is a somewhat schematic side view of the intraluminal device shown in FIG. 21, depicting the expansion member in the expanded state.
FIGS. 23A-23C illustrate sequentially performed aspects of the manner of operation and use of the intraluminal device shown in FIGS. 21 and 22 representing another example of the manner of operation and use disclosed here.
FIG. 24 illustrates another embodiment representing another example of the manner of operation and use disclosed here.
FIG. 25 is a cross-sectional view showing the blood vessel and the expanded expansion member in a deformed condition through the application of an outside pressing force.
FIGS. 26A-26D illustrate sequentially performed aspects of the manner of operation and use of the intraluminal device representing another example of the manner of operation and use disclosed here.
FIG. 27 is a cross-sectional view showing the blood vessel and the expanded expansion member in a deformed condition through the application of an outside pressing force.
FIGS. 28A-28D illustrate sequentially performed aspects of the manner of operation and use of the intraluminal device representing another example of the manner of operation and use disclosed here.
FIGS. 29A-29C are plan views of different configurations of the expansion member.

### DETAILED DESCRIPTION

Generally speaking, the device and method disclosed here are used to treat veins, including varicose veins. Varicose veins in the lower limb are most prevalent, though they also occur in pelvic and ovarian and spermatic cord veins. The treatment here seeks to close or occlude the affected vein. In one respect, this is accomplished by bringing a member into contact with the inner wall of the vein and moving the member along the vein that may cause a spasm that decreases the inner diameter of the vein (vein lumen). The member that is brought into contact with the inner wall of the vein is preferably an outwardly expandable member comprised of a plurality of contact members. The expansion member can be in the form of a spiral expansion member comprised of a plurality of spirally extending contact members. The device can be operated to expand the expanded expansion member from a first expanded state to a larger expanded state, and possible also vice versa. The damage to the vein can be further enhanced by applying an outside pressure force to the vein from outside the vein. This thus further urges the contact members of the expanded expansion member into strong (heavy) contact with the vein inner wall.

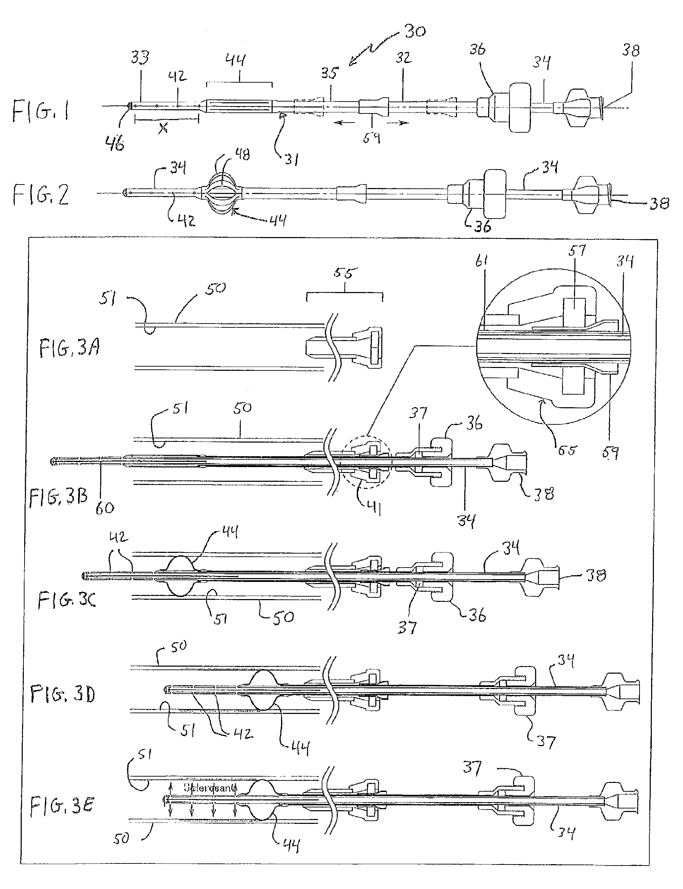

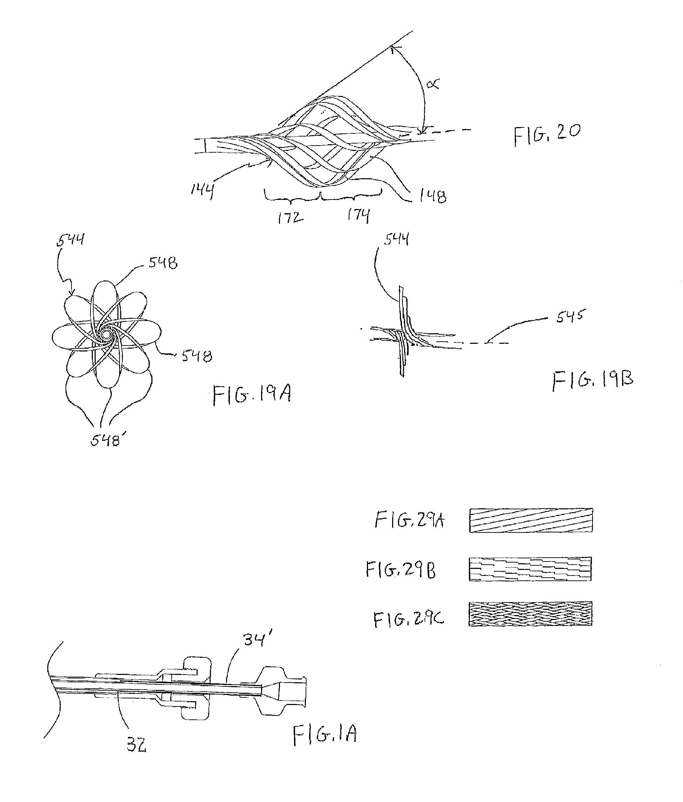

An intraluminal device (assembly) representing one example of the intraluminal device disclosed here is illustrated in FIGS. 1 and 2. Generally speaking, the intraluminal device 30 includes an elongated insertion portion 31 configured to be inserted into a blood vessel (varicose vein) of a living human body. The vein into which the insertion portion is inserted is a varicose vein for which treatment is required. The elongated insertion portion 31 includes a distal portion 33 possessing a circular cross-sectional shape and a proximal portion 35 also possessing a circular cross-sectional shape.

More specifically, the intraluminal device 30 includes an elongated outer sheath or outer member 32, an elongated inner tubular member or inner tube (inner member) 34, and an expandable expansion member 44. The proximal end of the elongated outer sheath 32 is connected to a stopper housing or hub (outer sheath hub) 36. The outer sheath 32 possesses a lumen, and the stopper housing 36 also possesses a lumen.

The elongated inner tube 34 is positioned inside the elongated outer sheath 32 and extends distally beyond both the distal end of the elongated outer sheath 32 and the distal end of the expandable expansion member 44, and also extends proximally beyond both the proximal end of the elongated outer sheath 32 and the proximal end of the stopper housing 36. The axial length (axial extent) of the elongated inner tube 34 (inclusive of the injection portion 38) exceeds the combined axial length of the elongated outer sheath 32, the expandable expansion member 44 and the stopper housing 36.

The elongated inner tube 34 is axially movable relative to the elongated outer sheath 32 and the stopper housing 37 (valve body). That is, the inner tube 34 is positioned in the lumen in the outer sheath 32 and the lumen in the stopper housing 36. The interior of the stopper housing 37 (the outer sheath hub 36) is preferably provided with a known stopper 37 as depicted in FIG. 3A. This stopper closes the lumen in the stopper housing 36 when the inner tube 34 is not positioned in the stopper housing 36. The stopper 37 is configured in a known manner to allow the inner tube 34 to pass through the stopper 37 while maintaining a liquid-tight seal between the stopper and the inner tube 34. Thus, when the elongated inner tube 34 is positioned inside the elongated outer sheath 32, the portion of the lumen on the distal side of the stopper 37 is sealed-off in a liquid-tight manner from the portion of the lumen on the proximal side of the stopper 37.

As described above, the inner tube 34 includes a lumen, and an injection port 38 is provided at (fixed to) the proximal end of the inner tube 34 and fluidly communicates with the lumen in the inner tube 34. The lumen in the inner tube 34 extends from the proximal-most end of the inner tube 34 in communication with the injection port 38, along the length of the inner tube 34 and up to the distal end portion of the inner tube 34. The distal end of the inner tube 34 is closed. The inner diameter of the lumen in the inner tube 34 at the distal end of the inner tube 34 is larger than the inner diameter of the lumen in the inner tube 34 at the proximal end of the inner tube 34. This can help contribute to rather easily injecting fluid in a smooth manner. Also, the inner diameter of the lumen in the inner tube 34 at the proximal end of the inner tube 34 is larger than the inner diameter of the lumen in the inner tube 34 at the distal end of the inner tube 34.

The distal end portion 40 of the inner tube 34 is provided with a plurality of axially and circumferentially spaced apart through holes 42. The through holes 42 are preferably equally spaced apart from one another, both axially and circumferentially. It is also possible though to vary the spacing between axially adjacent through holes 42 such that the axial spacing between the through holes 42 at the proximal portion of the through hole region X is greater than axial spacing between the through holes 42 at the distal portion of the through hole region X. Each of the through holes 42 communicates with the lumen in the inner tube 34. A fluid (e.g., a liquid such as a sclerosant or adhesive) is introduced into the injection port 38, flows along the lumen of the inner tube 34 and is ejected out of the holes 42 at the distal end portion 40 of the inner tube 34.

As mentioned above, the intraluminal device 30 also includes an expansion member 44. This expandable expansion member 44 is positioned between the proximal portion 35 of the elongated insertion portion 31 and the distal portion 33 of the elongated insertion portion 31. The expandable expansion member 44 possesses a proximal end fixed to the outer sheath 32 and a distal end fixed to the inner tube 34. In the illustrated embodiment, the proximal end of the expansion member 44 is fixed to the distal end of the outer sheath 32, while the distal end of the expandable expansion member 44 is fixed to the distal portion of the inner tube 34. In this illustrated embodiment, the expandable expansion member 44 is positioned proximally of all of the through holes 42 in the inner tube 34. That is, all of the through holes 42 are located distal of the distal-most end of the expandable expansion member 44. The through holes 42 are positioned in an axial region of the elongated inner tube 34 represented by the axial distance X in FIG. 1. That is, the axial distance X is the axial distance between the distal-most through hole(s) 42 and the proximal-most through hole(s) 42 in the elongated inner tube 34.

The expandable expansion member 44 is configured to be radially outwardly enlarged or expanded from the position illustrated in FIG. 1 to the position shown in FIG. 2. This outward radial expansion of the expansion member 44 occurs when the axial length of the expandable expansion member 44 is reduced (i.e., when the proximal and distal ends of the expansion member 44 are relatively moved to approach one another). In this illustrated embodiment, the expansion member 44 is radially outwardly expanded by relatively moving the inner tube 34 and the outer sheath 32. For example, holding the outer sheath 32 (stopper housing 36) stationary while axially moving the inner tube 34 in the proximal direction (i.e., to the right in FIG. 1) causes the distal end of the expandable expansion member 44 to also move axially in the proximal direction. This is because the expandable expansion member 44 is fixed to the distal portion of the inner tube 34 so that proximal movement of the inner tube 34 causes the expandable expansion member 44 to move with the inner tube 34. As the distal end of the expansion member 44 moves in the proximal direction towards the proximal end of the expansion member 44, the axial dimension of the expansion member 44 is reduced, and so the expansion member 44 expands radially outwardly so that the expansion member 44 is in an expanded state such as shown in FIG. 2. The degree or amount of radial outward expansion of the expandable expansion member 44 can be varied by adjusting the amount of relative axial movement between the proximal and distal ends of the expandable expansion member 44. That is, more axial movement of the inner tube 34 in the proximal direction relative to the outer sheath 32 will cause greater radial outward expansion of the expansion member 44, and less axial movement of the inner tube 34 in the proximal direction relative to the outer sheath 32 will cause less radial outward expansion of the expansion member 44.

The expandable expansion member 44 includes a plurality of expandable contact members. The contact members are best seen in FIG. 2 which illustrates the expanded expansion member 44 in the expanded state. The contact members 48 are configured so that in the expanded state, the expanded expansion member 44 includes a plurality of circumferentially spaced apart contact members 48. Thus, a space exists between each pair of circumferentially adjacent contact member 48, and each space opens to the interior region of the expansion member 44 that is surrounded by the expanded expansion member 44. In this embodiment, the contact members together form an expanded expansion member 44 whose overall outer shape is spherical. The overall outer shape of the expanded expansion member 44 will depend, at least in part, on the amount by which the expanded expansion member 44 is expanded. The contact members in this embodiment are parallel to the central axis 45 of the expanded expansion member 44 and the intraluminal device 30. Stated differently, the opposite ends of each contact member 48 are positioned along a line parallel to the central axis 45 of the expanded expansion member 44 and the intraluminal device 30.

The intraluminal device 30 can also include a marker 46 to facilitate locating the intraluminal device in a living body or blood vessel (e.g., vein). In the illustrated embodiment, the marker is provided at the distal-most end of the inner tube 34. The marker 46 can be made of radiopaque material that is visible under fluoroscopic observation. The marker 46 can also be made echogenic material that is visible under ultrasound. The intraluminal device 30 as well can be made of echogenic material that is visible under ultrasound.

The stopper 37 of the stopper housing 36 is disposed above the inner tube 34. After the expansion member 44 expands, the stopper 37 is secured to the inner tube 34.

The intraluminal device 30 illustrated in FIGS. 1 and 2 is used to treat varicose veins. FIGS. 3A-3D illustrate one manner of operation using the intraluminal device 30 illustrated in FIG. 1. Generally speaking, FIG. 3A illustrates an insertion aspect of the operation, FIG. 3B depicts an expansion aspect of the operation, FIG. 3C shows the abrasion aspect of the operation, and FIG. 3D illustrates the injection aspect of the operation.

To begin, the intraluminal device 30 is inserted into a blood vessel or vein of interest (e.g., an affected varicose vein). The intraluminal device 30 is then moved along the vein until reaching the target site or treatment location. FIG. 3B illustrates the intraluminal device located at the treatment site in a blood vessel or vein 50. As illustrated, the entire distal portion 33 of the elongated insertion portion 31, the entirety of the expandable expansion member 44, and at least a part of the proximal portion 35 of the elongated insertion portion 31 are positioned inside the blood vessel 50. The intraluminal device 30 is preferably positioned so that the expansion member 44 is positioned at the end (e.g., distal end) of the treatment location (i.e., the distal end of the part of the varicose vein to be treated). Or the expandable expansion member 44 is passed through the varicose vein to be treated and positioned at the distal end of the varicose vein or the vein (i.e., the distal end of the superficial vein including the varicose vein to be treated around the bifurcation between the deep vein and the superficial vein). As shown in FIG. 3A, the intraluminal device 30 is inserted into a blood vessel or vein via an introducer sheath. The introducer sheath has a valve in a proximal portion of the introducer sheath. While rotating and axially moving the expanded expansion member 44, the introducer sheath moves together with the expanded expansion member 44 due to the friction between the valve 57 and the outer sheath of the expanded expansion member. The friction should preferably be reduced or minimized to prevent the introducer sheath 55 from moving together with the elongated member. A proximal tube or slide tube 59 can be used at the proximal portion 61 of the outer sheath so that the friction between the valve 57 and the outer sheath is decreased or reduced.

Next, as illustrated in FIG. 3C, the expansion member 44 is radially outwardly expanded. In the preferred embodiment, this is accomplished by holding the outer sheath 32 (stopper housing 36) and pulling back or axially moving the inner tube 34 in the proximal direction (i.e., to the right in FIG. 3C). This causes the distal end of the expansion member 44 to also axially move in the proximal direction, towards the proximal end of the expansion member 44, so that the axial extent of the expansion member 44 is reduced, thus causing the radially outward expansion of the expansion member 44 as illustrated in FIG. 3C. A wire 60 is fixed the distal end of inner tube 34 in the inner tube 34 and is located the center of the inner tube 34. There are clearances between the wire 60 and an inner wall of the inner tube 34. When the proximal and distal ends of the expansion member 44 are relatively moved to approach one another, the wire 60 prevents the inner tube 34 inside the expansion member from breaking.

The expansion member 44 is expanded so that in the expanded state of the expansion member 44, the circumferentially spaced apart contact members 48 forming the expanded expansion member 44 directly contact the inner wall 51 of the vessel (vein) 50.

Next, as illustrated in FIG. 3D, the expanded expansion member 44 is moved axially along the blood vessel 50 in the proximal direction (i.e., to the right in FIG. 3D) while the contact members 48 remain in contact with the inner wall 51 of the blood vessel 50. The axial movement of the expanded expansion member 44 in the proximal direction is achieved by moving the entire intraluminal device 30 in the proximal direction. After a step of inserting the intraluminal device 30, the expandable expansion member 44 can be passed through the varicose vein and positioned at the distal end of the varicose vein or the vein. After the expandable expansion member expands at the distal end of the varicose vein or the vein, while the intraluminal device is withdrawn, the expanded member passes along or moves along the varicose vein to be treated. The step of passing can be repeated from the distal end to the proximal end or from the proximal end to the distal end. The movement of the entire intraluminal device 30 as described above means that the inner tube 34, the outer sheath 32, the housing 36, and the expansion member 44 are all axially moved together in the proximal direction. During this axial movement, the relative position of the inner tube 34 relative to the outer sheath 32, preferably remains constant. That is, the inner tube 34 is preferably not moved relative to the outer sheath 32. Suitable features can be provided to help maintain the relative position of the inner tube 34 relative to the outer sheath 32.

The contact of the contact members 48 with the inner wall 51 of the blood vessel 50 causes damage to the blood vessel 50. This contact means that the vein or blood vessel 50 may experience spasm, causing the inner diameter of the vein 50 to decrease, preferably in a uniform manner.

It is possible during the axial movement of the expanded expansion member 44 in the proximal direction to also rotate the expanded expansion member 44, thus creating more contact with, and damage to, the inner wall 51 of the blood vessel 50. That is, rotating and axially moving the expanded expansion member 44 while the contact members 48 are in contact with the inner wall 51 of the vessel 50 causes the contact members to contact a larger area or surface of the inner wall of the vessel than might otherwise be the case. While rotating and axially moving the expanded expansion member 44, twisting of the vein can occur by rotating the expanded spiral expansion member so that the diameter of the vein decreases (decreasing the diameter of the vein enhances damage to the inner wall of the vein).

Following the axial movement of the expanded expansion member 44 along a portion of the vessel 50, the axial movement of the expanded expansion member 44 is stopped. Then, as illustrated in FIG. 3E, a fluid (e.g., a liquid such as a sclerosant or adhesive) is introduced into the injection port 38, flows along the lumen of the inner tube 34 and is ejected out through the through holes 42 in the inner tube 34. This fluid enters the blood vessel 50 in the region that has just been in contact with, and damaged by the expanded expansion member 44. This fluid is also directed towards the inner wall 51 of the vessel 50. Sclerosant is a preferred fluid as it is an injectable irritant that causes inflammation and subsequent fibrosis to close off the lumen in the blood vessel 50. After a desired amount of fluid has been ejected into the vessel 50, the supply of fluid to the injection port 38 is stopped to stop ejecting fluid through the through holes 42 of the inner tube 34 and into the vessel 50. The plurality of through holes is arranged in the circumferential direction of the elongated inner member. This helps facilitate the injection of fluid toward the inner wall of the vein. The plurality of through holes is also arranged in the same direction as the plurality of contact members of the expanded spiral expansion member. This helps make it easier to uniformly inject fluid toward the damaged area. The expanded expansion member is positioned in the center of the blood vessel (vein). Thereby, the elongated inner member is positioned in the center of the blood vessel (vein). This helps make it easier to uniformly inject fluid toward the damaged area/ the varicose vein.

When the supply of fluid to the interior of the vessel 50 is stopped, the expanded expansion member 44 is once again axially moved in the proximal direction while the contact members 48 of the expanded expansion member 44 remain in contact with the inner wall 51 of the vessel 50 in the manner described above to damage the next axial portion of the vessel 50 to be treated. The axial movement of the expanded expansion member 44 is then stopped, and the fluid is once again supplied to the injection port 38 and ejected through the through holes 42 toward the inner wall 51 of the vessel 50 and into the vessel as discussed above. This operational sequence, involving axially moving the expanded expansion member 44 while the contact members 48 of the expanded expansion member 44 remain in contact with the inner wall 51 of the vessel 50, stopping the axial movement of the expanded expansion member 44 while the contact members 48 of the expanded expansion member 44 remain in contact with the inner wall 51 of the vessel 50, ejecting fluid into the blood vessel while the contact members 48 of the expanded expansion member 44 remain in contact with the inner wall 51 of the vessel 50, and then stopping the ejection of the fluid into the vessel 50 while the contact members 48 of the expanded expansion member 44 remain in contact with the inner wall 51 of the vessel 50 is repeated until the entire axial extent of the vein to be treated has been treated.

As discussed above, the operation of the intraluminal device involves expanding the expansion member 44 into contact with the inner wall 51 of the blood vessel 50, axially moving the expanded expansion member 44 in the proximal direction over a desired axial distance, stopping the axial movement of the expanded expansion member 44, ejecting fluid into the vein, etc. This desired axial distance is preferably related to the axial dimension X over which the region of through holes 42 extends. The desired axial distance along which the expanded expansion member 44 moves between each start and stop of axial movement is preferably 0.5X - 1.5X, more preferably 0.8X - 1.2X. In this way, the expanded expansion member 44 contacts and damages the inner wall of the blood vessel 50 over an axial extent that generally corresponds to the axial extent of the region covered by the through holes 42 in the inner tube 34, which is also the axial region of the inner wall 51 of the vessel 50 covered by the fluid (e.g., liquid such as a sclerosant) that is ejected through the through holes.

The embodiment of the intraluminal device described above is particularly advantageous as it allows the user to use a single hand to axially move the inner tube using one hand and injecting the fluid using the same hand. The intraluminal device allows the user to use a single hand to both axially move the inner tube and inject the fluid. The intraluminal device thus makes it possible for the user to use one hand to axially move the inner tube and inject the fluid, while using the other hand for other aspects of the operation such as ultrasound observation.

As shown in FIG. 1A, the intraluminal device illustrated in FIG. 1 can be modified so that the proximal portion of the shaft of the inner tube 34 possesses a hard member 34' (for example a member made of stainless steel or a plastic material harder than the inner tube). This hard member helps facilitate sliding relative to the inner surface of the proximal portion of the outer sheath 32 so that relatively smooth sliding surfaces are provided.

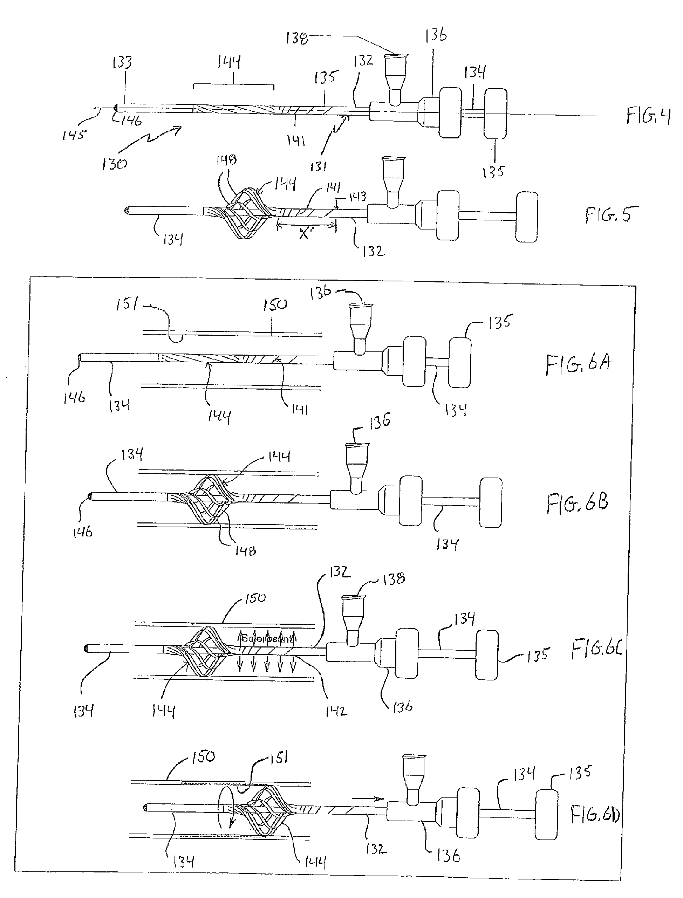

FIGS. 4, 5 and 6A-6D illustrate an embodiment of the intraluminal device 130 representing another example of the intraluminal device and operational method disclosed here. Features in this embodiment which are similar to features described in the earlier embodiment are identified by common reference numerals, but preceded by a "1". A detailed description of features in this embodiment that are similar to features in the earlier embodiment is not repeated. The following detailed description focuses primarily on differences between this embodiment and the earlier embodiment.

In this embodiment shown in FIGS. 4 and 5, the injection port 138 forms a part of the stopper housing (outer sheath hub) 136. The injection port 138 fluidly communicates with the lumen in the outer sheath 132. Fluid (e.g., sclerosant or adhesive) injected into the injection port 138 flows into the lumen in the outer sheath 132. That is, by virtue of the injection port 138 being in fluid communication with the lumen in the outer sheath 132, fluid entering the injection port 138 flows into the lumen in the outer sheath 132 (i.e., in the space between the inner surface of the outer sheath 32 and the outer surface of the inner tube 34).

In the first embodiment described above, through holes 42 were provided in the inner tube 34 to eject the fluid into the blood vessel. In this embodiment shown in FIGS. 4 and 5, the through holes 142 are provided in the outer sheath 132 and fluidly communicate with the lumen in the outer sheath 132.

In the illustrated embodiment of the intraluminal device shown in FIGS. 4 and 5, the through holes 142 in the outer sheath 132 are circumferentially and axially spaced apart spaced apart, and are arranged along respective spiral paths as illustrated by the spiral arrangement 141 in FIGS. 4 and 5. The through holes 142 are positioned between the proximal end of the expandable expansion member 144 and the proximal end of the outer sheath 132.

In this embodiment, a handle 135 is also fixed to the proximal end of the inner tube 134. This helps facilitate operation and axial movement of the inner tube 134.

The expandable expansion member 144 in the embodiment of the intraluminal device 130 shown in FIGS. 4 and 5 is a spiral expansion member 144. That is, the contact members forming the expandable expansion member 144 are spiral-shaped contact members. This can be seen in FIG. 5 illustrating the expansion member 144 in an expanded state in which the expansion member 144 is radially outwardly expanded. Each of the individual spirally extending contact members (spiral-shaped contact members) 148 forming the spiral expansion member 144 extends in a spiral manner about the axis of the expansion member 144. The individual contact members 148 are circumferentially spaced apart from one another in the manner illustrated so that a space exists between each circumferentially adjacent pair of contact members 148.

The spiral arrangement of the through holes 142 in the outer sheath 132 are spirally arranged in a spiral direction opposite the spiral direction of the spiral expansion member 144. This helps maintain the outer diameter of the outer sheath 132. That is, spirally arranging the spiral arrangement of through holes 142 and the spiral expansion member 144 in opposite directions means that the outer diameter of the outer sheath need not be increased to accommodate both the spiral arrangement of the through holes 142 and the spiral expansion member 144. If the spiral direction of the through holes 142 was the same as the spiral direction of the spiral expansion member 144, the strength of the outer sheath might be reduced or compromised, and the outer sheath might break. Spirally arranging the spiral arrangement of through holes 142 and the spiral expansion member 144 in opposite directions helps avoid such concerns. Also, the distance between axially adjacent spirals of the through holes forming the spiral arrangement of through holes 142 is greater towards the proximal end of the outer sheath as shown in FIGS. 4 and 5. More specifically, the spacing or distance between axially adjacent spirals becomes gradually larger towards the proximal end of the outer sheath. The stiffness or rigidity of the outer sheath 132 in the area of the spiral arrangement of through holes 142 thus increases.

The spirally extending contact members 144 in this embodiment are not parallel to the central axis 145 of the expansion member 144 and the intraluminal device 130. Stated differently, the opposite ends of each spirally extending contact members 148 are positioned along a line not parallel to (transverse to) the central axis 145 of the expansion member 144 and the intraluminal device 130.

FIGS. 6A-6D illustrate the operation or manner of use of the intraluminal device shown in FIGS. 4 and 5. The intraluminal device 130 is initially placed in a blood vessel (varicose vein) and advanced along the vessel until the expandable spiral expansion member 144 is located at the treatment site as illustrated in FIG. 6A. The intraluminal device is positioned so that the distal end of the spiral expansion member 144 is located at the distal end of the region of the blood vessel 150 to be treated.

Next, as illustrated in FIG. 6A, the spiral expansion member is expanded to an expanded state in which the expanded spiral expansion member 144 is in contact with the inner wall 151 of the blood vessel 150. That is, the spirally extending contact members 148 directly contact the inner wall 151 of the vessel 150. Next, fluid (e.g., a liquid such as sclerosant or adhesive) is introduced or injected into the injection port 138. This fluid flows into the lumen of the outer sheath 132 and is ejected through the through holes 142 in the outer sheath 132 into the vessel 150 and toward the inner wall of the vessel 150 as illustrated in FIG. 6C.

After the desired amount of fluid is ejected into the blood vessel, the supply of fluid to the injection port 138 is stopped, and so the ejection of the fluid into the blood vessel by way of the through holes 142 stops. At this time, the expanded spiral expansion member 144 is axially moved along the vessel 150 in the proximal direction while the spirally extending contact members 148 remain in direct contact with the inner wall of the vessel 150. As explained above, this can be accomplished by axially moving the entire intraluminal device 130 in the proximal direction (i.e., to the right in FIG. 6D).

In this version of the intraluminal device, the spiral configuration of the spirally extending contact members 148 forming the expanded spiral expansion member 144 automatically causes the expanded spiral expansion member 144 to rotate about its central axis. That is, by virtue of the spiral configuration of the contact members 148 and the contact of the expanded spiral expansion member 144 with the inner wall of the blood vessel during the axial movement of the expanded spiral expansion member 144, the expanded spiral expansion member 144 automatically rotates as the expanded spiral expansion member 144 moves axially. In this embodiment of the intraluminal device, the entire intraluminal device 130 rotates.

The through holes 142 in the outer sheath 132 extend from a position close to the expansion member 144 and terminate at a point 143 representing the proximal end of the through hole region. The distance X' shown in FIG. 5 represents the distance from the proximal-most end of the expanded spiral expansion member 144 and the proximal end 143 of the through hole region.

In a manner similar to that discuss above, sclerosant is ejected from the through holes 142 over a region identified by the distance X' in FIG. 5. After the fluid (sclerosant or adhesive) is ejected into the vessel 150, the expanded spiral expansion member 144 is axially moved over a distance 0.5X' - 1.5 X', more preferably 0.8 X' - 1.2 X'.

The operational sequence described above, involving ejecting fluid into the vessel 150 while the spirally extending contact members 148 of the expanded spiral expansion member 144 remain in contact with the inner wall 151 of the vessel 150, stopping the ejection of the fluid into the vessel 150, axially moving the expanded spiral expansion member 144 while the contact members 148 of the expanded spiral expansion member 144 remain in contact with the inner wall 151 of the vessel 150, and then stopping the axial movement of the expanded spiral expansion member 144 while the spirally extending contact members 148 of the expanded spiral expansion member 144 remain in contact with the inner wall 151 of the vessel 150 is repeated until the entire axial extent of the vein to be treated has been treated.

The embodiment of the intravascular device illustrated in FIGS. 4-6 can include an arrangement of through holes in the inner tube 132 similar to the through holes 42 in the embodiment shown in FIGS 1-3. Similarly, the embodiment of the intraluminal device illustrated in FIGS. 1-3 can be modified to include the arrangement of through holes in the outer sheath as illustrated in FIGS. 4-6 rather than the inner tube. It is also possible to utilize the expandable spiral expansion member 144 in the intraluminal device shown in FIGS. 1 and 2, and to utilize the expandable expansion member 44 in the intraluminal device shown in FIGS. 4 and 5.

FIG. 20 illustrates the expanded spiral expansion member 144 configured so that respective spaces exist between circumferentially adjacent spirally extending contact members 148.

Using the embodiment of the intraluminal device 130 shown in FIGS 4, 5 and 6A-6D, it is possible to axially move and rotate the expanded expansion member 144 with one hand, while also injecting fluid at the injection port using the same hand. The injection of fluid at the injection port can occur using a syringe.

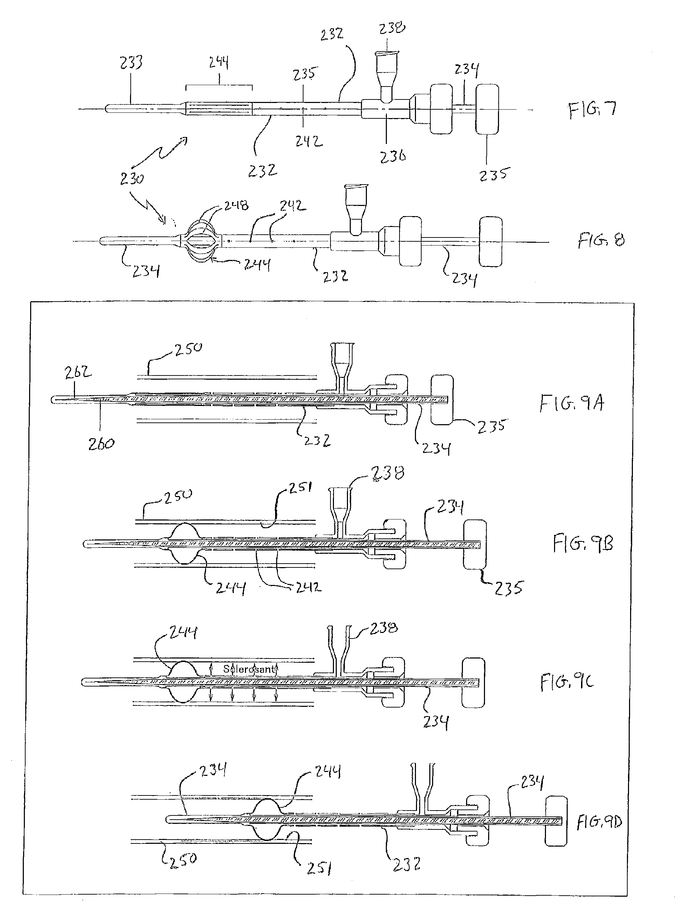

FIGS. 7, 8 and 9A-9D illustrate an embodiment of the intraluminal device 230 representing another example of the intraluminal device and operational method disclosed here. Features in this embodiment which are similar to features described in earlier embodiments are identified by common reference numerals, but preceded by a "2". A detailed description of features in this embodiment that are similar to features in the earlier embodiment is not repeated. The following detailed description focuses primarily on differences between this embodiment and the second embodiment shown in FIGS. 4-6.

This third embodiment shown in FIGS. 7-9 differs from the second embodiment in that the through holes 242 in the outer sheath are not arranged in a spiral manner. Rather, the through holes 142 are arranged in rows extending along (i.e., parallel to) the longitudinal extent or central axis of the outer sheath 232. In addition, the contact members 248 forming the expandable expansion member 244 are not spirally extending contact members, but rather are contact members like those shown in FIG. 2 that extend along (i.e., are parallel to) the longitudinal extent or central axis of the intraluminal device.

Further, FIGS. 9A-9D illustrate that the inner tube 234 in this embodiment is in the form of an elongated member comprised of a core wire 260 and an outer layer 262 of resin covering the core wire 260. In the illustrated example, the outer resin layer 262 covers the core wire along the entire axial extent of the core wire 260.

This embodiment of the intraluminal device, the inner tube 234 comprised of the core wire 260 with the outer resin layer 262 is a guide wire.

The manner of operation or use of the intraluminal device illustrated in FIGS. 7-9 is the same as that described above with respect to embodiment shown in FIGS. 4-6, and so a detailed description of the manner of operation of this third embodiment will not be repeated here.

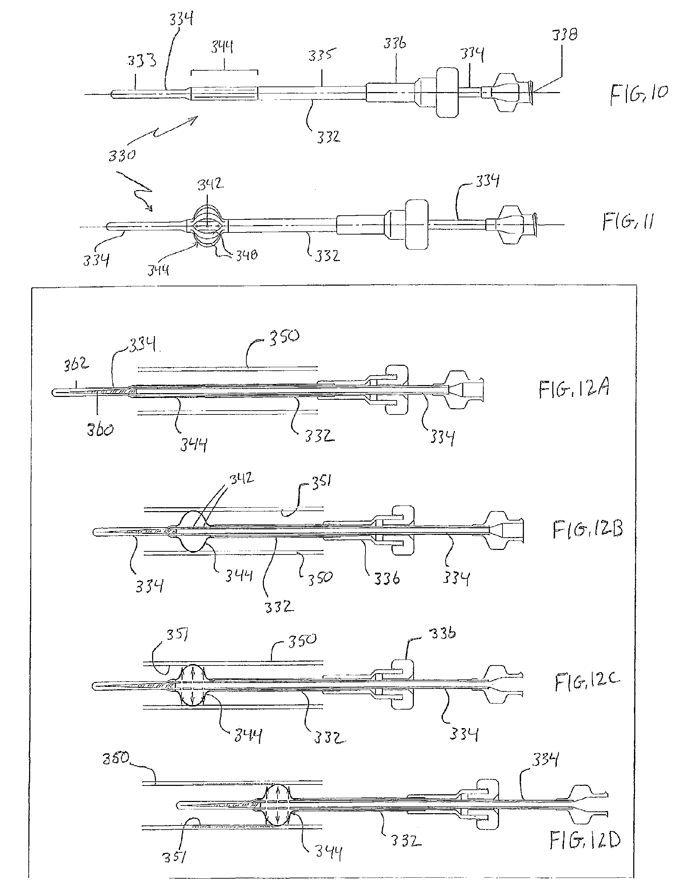

FIGS. 10, 11 and 12A-12D illustrate an embodiment of the intraluminal device 330 representing another example of the intraluminal device and operational method disclosed here. Features in this embodiment which are similar to features described in the earlier embodiment are identified by common reference numerals, but preceded by a "3". A detailed description of features in this embodiment that are similar to features in earlier embodiments is not repeated. The following detailed description focuses primarily on differences between this embodiment and the first embodiment shown in FIGS. 1-3.

The embodiment shown in FIGS. 10, 11 and 12A-12D is similar to the embodiment illustrated in FIGS. 1-3, except primarily for the location of the through holes in the elongated inner member. In the embodiment illustrated in FIGS. 10-12, the through holes 342 are positioned in a portion of the inner tube 334 that is surrounded by the expandable expansion member 344. Thus, the region of the inner tube 334 containing the through holes 342 axially overlaps with the expandable expansion member 344.

In this embodiment, the distal end portion of the inner tube 334 includes the core wire 360 and the resin coating 362. The remainder of the inner tube 334 is hollow so that the lumen extends along the axial extent of the inner tube 334 up to the core wire. The lumen in the inner tube 334 communicates with the injection port 338. The through holes 342 in the inner tube 334 are in fluid communication with the lumen in the inner tube 334.

FIGS. 12A-12D illustrate a manner of use or operation of the intraluminal device 330 illustrated in FIGS. 10 and 11. First, the device is positioned in a blood vessel (e.g., an affected varicose vein) as illustrated in FIG. 12A. The device 330 is moved along the vessel 350 until the expandable expansion member 344 is located at the distal end of the portion of the vein 350 to be treated. Then, as illustrated in FIG. 12B, the expandable expansion member 344 is outwardly expanded to an expanded state. This is accomplished in a manner similar to that described above, so that the contact members 348 forming the expanded expansion member 344 directly contact the inner wall 351 of the vessel 350. Next, fluid (e.g., a sclerosant or adhesive) is injected into the lumen in the inner tube 334 by way of the injection port 338. The fluid flows through the lumen in the inner tube 334 and is ejected through the through holes 342 and toward the inner wall 351 of the vessel so as to be introduced into the vessel as illustrated in FIG. 12C. The expanded expansion member 344 is then moved axially along the length of the vessel 150 while the fluid continues to be ejected through the through holes 342. Thus, the axial movement of the expanded expansion member 344 and the ejection of the fluid toward the inner wall of the vessel occur simultaneously. With this embodiment, it is thus not necessary to stop the axial movement of the expanded expansion member 344. Nor is it necessary to stop ejecting the fluid. During the axial movement of the expanded expansion member 344, it is also possible to rotate the expanded expansion member. Combining axial movement of the expanded expansion member 344 with rotation of the expanded expansion member may help further damage the inner wall 352 of the vessel 350 and facilitate the treatment described above. It is also possible here to use an expandable spiral expansion member similar to the expandable spiral expansion member 144 described above so that the spirally extending contact members forming the expanded spiral expansion member automatically cause the expanded spiral expansion member to rotate about its central axis.

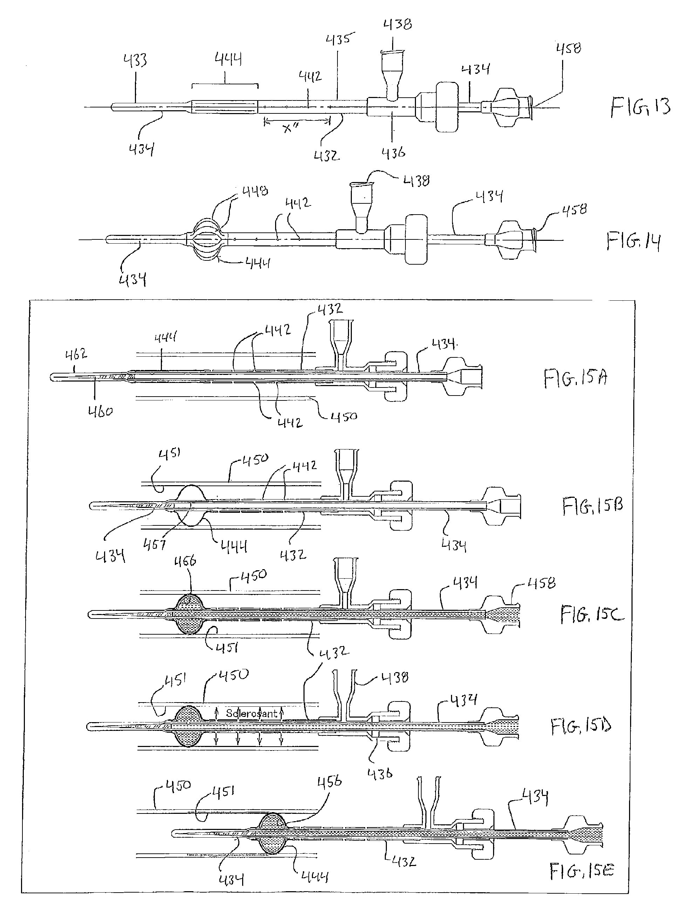

FIGS. 13, 14 and 15A-15E illustrate an embodiment of the intraluminal device 430 representing another example of the intraluminal device and operational method disclosed here. Features in this embodiment which are similar to features described in the earlier embodiment are identified by common reference numerals, but preceded by a "4". A detailed description of features in this embodiment that are similar to features in earlier embodiments is not repeated. The following detailed description focuses primarily on differences between this embodiment and the third embodiment shown in FIGS. 7, 8 and 9A-9D.

As shown in FIGS. 13 and 14, this embodiment of the intraluminal device 430 includes the injection port 438 that communicates with the lumen in the outer sheath 432 to deliver fluid (e.g., sclerosant or adhesive) to the through holes 442 in the outer sheath 432. In addition, as best illustrated in FIGS. 15A-15E, a balloon 456 is positioned inside the expandable expansion member 444. The axial extent of the balloon 456 is generally the same as the axial extent of the expandable expansion member 444. The distal end of the balloon 456 is fixed to the elongated inner tube 434, and the proximal end of the balloon 456 is fixed to the distal end of the elongated outer sheath 432. The lumen in the elongated inner tube 434 communicates with another injection port 458 at the proximal end of the elongated inner member 434. In addition, the inner tube 434 is provided with one or more through holes 457 that communicate with the interior of the balloon 456. Fluid introduced into the injection port 458 enters the lumen in the elongated inner tube 434, passes through the through hole(s) 457 and enters the balloon 456 to radially outwardly expand the balloon as generally illustrated in FIG. 15C. When the expandable expansion member 444 is in the non-expanded condition shown in FIG. 13, the balloon 456 is contracted/deflated.

Referring to FIGS. 15A-15E, set forth next is a description of the manner of use and operation of the intraluminal device 430 illustrated in FIGS. 13 and 14. The intraluminal device 430 is initially positioned in a vessel 450, and the expandable expansion member 444 is outwardly expanded in the manner described above as generally illustrated in FIG. 15B. This causes the contact member 448 forming the expanded expansion member 444 to directly contact the inner wall 451 of the vessel 450.

Next, as illustrated in FIG. 15C, fluid (e.g., saline) is introduced into the injection port 458 to radially outwardly expand the balloon 456. This outward expansion of the balloon 456 applies a force to the already expanded expansion member 444 to ensure contact of the expanded expansion member 444 with the inner wall 451 of the vessel 450. The balloon 456 remains in an inflated or expanded state pressing against the expanded expansion member 444 throughout subsequent axial movement of the expanded expansion member 444. This helps ensure that the desired degree of contact occurs between the expanded expansion member 444 and the inner wall 451 of the vein 450 so that appropriate damage is imparted to the vein.

As illustrated in FIG. 15D, after the balloon is expanded into contact with the expanded expansion member 444, fluid (e.g., liquid such as sclerosant or adhesive) is injected into the port 438. This fluid enters the lumen in the outer sheath 432 and is ejected through the through holes 442 in the outer sheath. The fluid is directed at the inner wall 451 of the vessel 450 and enters the vessel. Next, as illustrated in FIG. 15E, the expanded expansion member 444 is moved axially in the proximal direction (to the right in FIG. 15E) while the expanded expansion member 444 remains in contact with the inner wall 451 of the vessel 450. As mentioned above, during this axial movement of the expanded expansion member 444, the balloon 456 remains in the expanded state and in contact with the expanded expansion member 444 to ensure continued and ongoing direct contact of the expanded expansion member 444 with the inner wall 451 of the vessel 450. After a desired amount of fluid is ejected toward the inner wall of the vessel 450 and into the vessel 450, The ejection of the fluid is stopped. After the fluid ejection has stopped, the expanded expansion member 444, pressed outwardly by the inflated balloon 456, is axially moved along the vessel 450 to cause damage to the vessel as described above.

The expanded expansion member 444 is preferably axially moved over a distance equal to 0.5X" - 1.5 X", more preferably 0.8 X" - 1.2 X". This distance represents the distance between the proximal-most and distal-most through holes 442 (i.e., the axial dimension of the region containing the through hole 442). This generally corresponds to the portion of the inner wall 451 of the vessel 450 covered by the fluid ejected through the through holes 452 when the expanded expansion member 444 is stationary.

The operational sequence described above, involving ejecting fluid into the vessel 450 while the contact members 448 of the expanded spiral expansion member 444 remain in contact with the inner wall 451 of the vessel 450, stopping the ejection of the fluid into the vessel 450, axially moving the expanded expansion member 444 while the contact members 448 of the expanded expansion member 444 remain in contact with the inner wall 451 of the vessel 450, and then stopping the axial movement of the expanded expansion member 444 while the contact members 448 of the expanded expansion member 444 remain in contact with the inner wall 451 of the vessel 450 is repeated until the entire axial extent of the vessel to be treated has been treated.

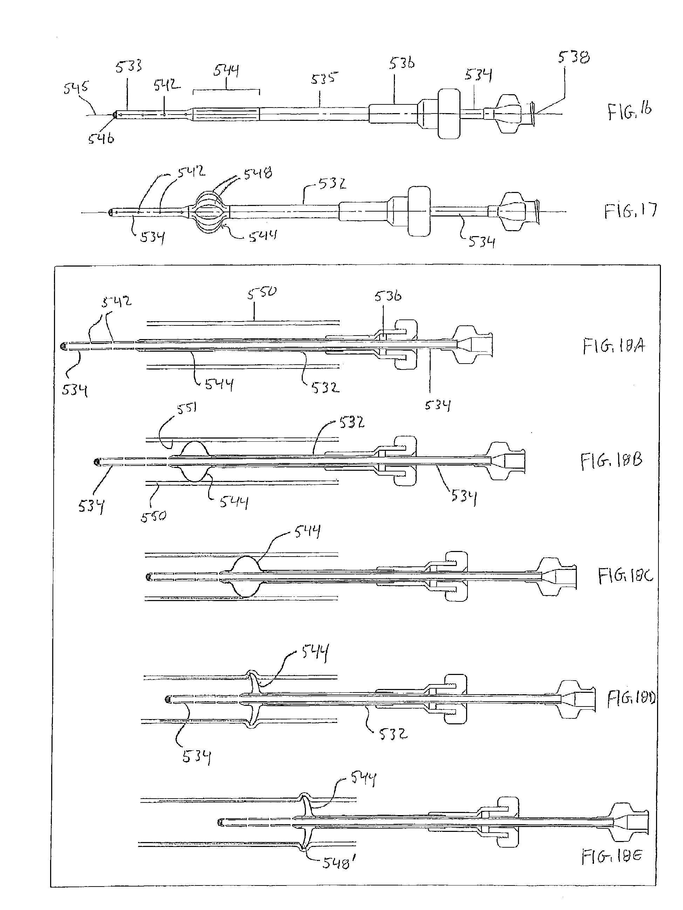

FIGS. 16, 17 and 18A-18E illustrate an embodiment of the intraluminal device 530 representing another example of the intraluminal device and operational method disclosed here. Features in this embodiment which are similar to features described in the earlier embodiment are identified by common reference numerals, but preceded by a "5". A detailed description of features in this embodiment that are similar to features in earlier embodiments is not repeated. This embodiment of the intraluminal device shown in FIGS. 16 and 17 is the same as the first embodiment shown in FIGS. 1 and 2 and so a detailed description of the features associated with the intraluminal device is not repeated. The following detailed description focuses primarily on differences between the manner of use or operation of this this embodiment and the manner of use or operation of the first embodiment shown in FIGS. 3A-3D.

The use and operation of the embodiment of the intraluminal device shown in FIGS. 16 and 17 is illustrated in FIGS. 18A-18E.

To start, the intraluminal device is inserted into a vein as illustrated in FIG. 18A, and then the expandable expansion member 544 is radially outwardly expanded to a first expansion state in the manner described above. In this first expansion state, the expanded expansion member 544 is in contact with the inner wall 551 of the vessel 550 (varicose vein) as shown in FIG. 18B. That is, in the first expansion state, the contact members 548 forming the expanded expansion member 544 directly contact the inner wall 551 of the vessel 550.

The expanded expansion member 544 is then axially moved along a portion of the vein 550 as described above to damage the blood vessel as generally illustrated in FIG. 18C. The axial movement of the expanded expansion member 544 is then stopped. Fluid (e.g., a liquid such as sclerosant or adhesive) can then be ejected into the vessel 550 toward the inner wall 551 of the vessel by way of the through openings 542 in the distal end portion of the inner tube 534 in a manner similar to that described above.

Next, as illustrated in FIG. 18D, the expansion member 544 which has been expanded to the first expansion state is further expanded to a second expansion state. The outer dimensions (outer diameter) of the further expanded expansion member expanded to the second expansion state as illustrated in Fig. 18D is larger than the outer dimensions (outer diameter) of the expanded expansion member 544 expanded to the first expansion state shown in FIG. 18C.

Expanding the expansion member 544 from the first expansion state to the larger second expansion state can be beneficial when moving the expanded expansion member 544 along a vessel (varicose vein) whose inner diameter is increasing. In such a situation, by further expanding the expanded expansion member from the first expansion state to the larger second expansion state, it is possible to maintain contact with the inner wall 551 of the blood vessel 550 to ensure appropriate damage to the vein. Thus, as the expanded expansion member 544 moves axially along the vessel 550 from a portion of the vessel 550 having a first inner diameter to a portion of the vein having a second larger inner diameter, the expanded expansion member 544 can be further radially outwardly expanded so that the expanded expansion member 544 becomes a further expanded expansion member 544. This further expansion of the expanded expansion member 544 from the first expansion state to the larger second expansion state can be achieved in the same way as the expansion of the expansion member from the non-expanded state shown in FIG 18A to the first expanded state shown in FIG. 18A as described above (by axially moving the inner tube 534 in the proximal direction (i.e., to the right in FIG. 18C).

It is also possible to reduce the expansion of the expanded expansion member 544 from a first outer dimension (outer diameter) to a second smaller outer dimension (second smaller outer diameter) by axially moving the inner tube 534 in the distal direction (i.e., to the left in FIGS. 18A-18E).

After the expansion member 544 is expanded to the second expanded state shown in FIG. 18D, the further expanded expansion member 544 is axially moved along the vein as illustrated in FIG. 18E. During this movement, the peaks 548' of the contact members 548 forming the further expanded expansion member 544 are in contact with the inner wall 551 of the vein 550 as illustrated in FIGS. 18D and 18E and so the contact members 548 tend to bend or curve as shown in FIGS. 18D and 18E. The further expanded expansion member 544 thus takes on a concave shape.

In the embodiment illustrated in FIGS. 16-18, the contact numbers 548 extend generally parallel to the axis of the device as generally shown in FIG. 17. It is also possible to replace the expandable expansion member shown in FIGS. 15 and 17 with the expandable expansion member shown in FIGS. 4 and 5 that is comprised of a plurality of spirally extending contact members. In this case, when the expanded expansion member is radially outwardly expanded from the first expansion state to the second expansion state, the further expanded expansion member 544 possesses a side view similar to that shown in FIGS. 18D and 18E. But from the distal end of the device, the further expanded expansion member possesses the configuration shown in FIG. 19A in which the further expanded expansion member includes a plurality of spiral contact members each having tops or peaks in contact with the inner wall of the vessel. The spiral contact members each form a loop, and the loops are all arranged in a plain perpendicular to the longitudinal axis 545 of the intraluminal device. A part of each of the loops overlaps a circumferentially adjacent loop as shown in FIG. 19A. FIG. 19B is a side view of a portion of the further expanded expansion member shown in FIG. 19A, but illustrating the arrangement of the loops in a plane perpendicular to the longitudinal axis 545 of the further expanded expansion member 544 (i.e., from the side).

Referring to FIG. 20, this illustrates an example of the expanded spiral expansion member in the first expanded state. Here, respective spaces exist between circumferentially adjacent spirally extending contact members 148. The size of the spaces increases as the expansion member is further expanded outwardly. Thus, the spacing between circumferentially adjacent spirally extending contact members of the expanded spiral expansion member in the second expanded state is greater than the spacing between circumferentially adjacent spirally extending contact members of the expanded spiral expansion member in the first expanded state.

Referring still to FIG. 20, the expanded spiral expansion member 144 possesses one part 172 (the distal half of the expanded spiral expansion member 144) and an other part 174 (the proximal half of the expanded spiral expansion member 144). When the inner surface of the one part contacts the inner surface of the other part, the expanded spiral expansion member can damage the inner wall of the vein more strongly because these two parts are in contact with each other and help maintain strong contact with the inner wall.

The spirally extending contact members 148 form an angle α with the horizontal axis. This angle α makes it relatively easy to rotate the expanded expansion member. The angle α can be from 10° to 80°, preferably 20° to 70°, more preferably 30° to 80°.

When the expansion member 544 is moved from the first expanded state shown in FIGS. 18B and 18C to the second expansion state illustrated in FIGS. 18D and 18E, the length of the expansion member 544 along the longitudinal axis of the expansion member is reduced, and the opposite axially ends of the expansion member 544 approach one another. When the expansion member 544 is in the second expanded state illustrated in FIGS. 18D, 18E, 19A and 19B, the inner surface of the one part 172 of the expansion member contacts the inner surface of the other part of the expansion member 174. As illustrated in FIG. 20, the inner surfaces of the one part 172 and the other part 174 of the expansion member 144 are spaced apart from one another when the expansion member is in the first expansion state.

FIG. 19A illustrates that when spirally extending contact members 544 are in the second expanded state the loops possess peaks 548', and the peaks are circumferentially spaced apart from one another, though circumferentially adjacent loops overlap one another.

After the expansion member 544 is further expanded to the second expansion state illustrated in FIG. 18D and 18E, the further expanded expansion member 544 is axially moved along the vessel using one hand. This axial movement of the further expanded expansion member causes the further expanded expansion member 544 in the second expansion state to rotate by virtue of the loops of the further expanded expansion member 544.

It is possible during use of the assembly or intraluminal device disclosed here that as the device is being moved along the vessel, the expansion member will contact or catch on the valve ring in the vessel (varicose) vein. Several techniques can be employed, individually or successively, to detach or separate the expansion member 544 from the valve ring. For example, it is possible to deform (contract) the expansion member 544 from the second expanded state (larger expansion state) to the first expanded state (smaller expansion state). To detach the expanded expansion member 544 from the valve ring, it is also possible to rotate the expanded expansion member 544 and/or repeatedly move the expanded expansion member axially back and forth. It is also possible during use of the assembly or intraluminal device disclosed here that as the device is being moved along the vessel, the expansion member might damage the valve ring in the vessel (varicose vein). Several techniques can be employed, individually or successively, to avoid destroying the function of the valve and/or damaging the valve ring. For example, it is possible to rotate the expanded expansion member distal of the valve ring and/or proximal of the valve ring. While the expanded expansion member rotates, the expanded expansion member can pass the valve/valve ring both in the distal/ proximal direction. While the expanded expansion member rotates, the expanded expansion member contacts the distal/proximal side of the valve/valve ring. It is also possible to repeat the rotation and the opposed rotation of the expanded expansion member for distal of valve ring and/or proximal of the valve ring. It is also possible to vibrate the expanded expansion backward and forward for distal of valve ring and/or proximal of the valve ring. The function of the valve ring can be destroyed and it is easy for fluid to flow so that the damaged vein or the damaged varicose vein by the expanded expansion member can be injected fluid certainly.

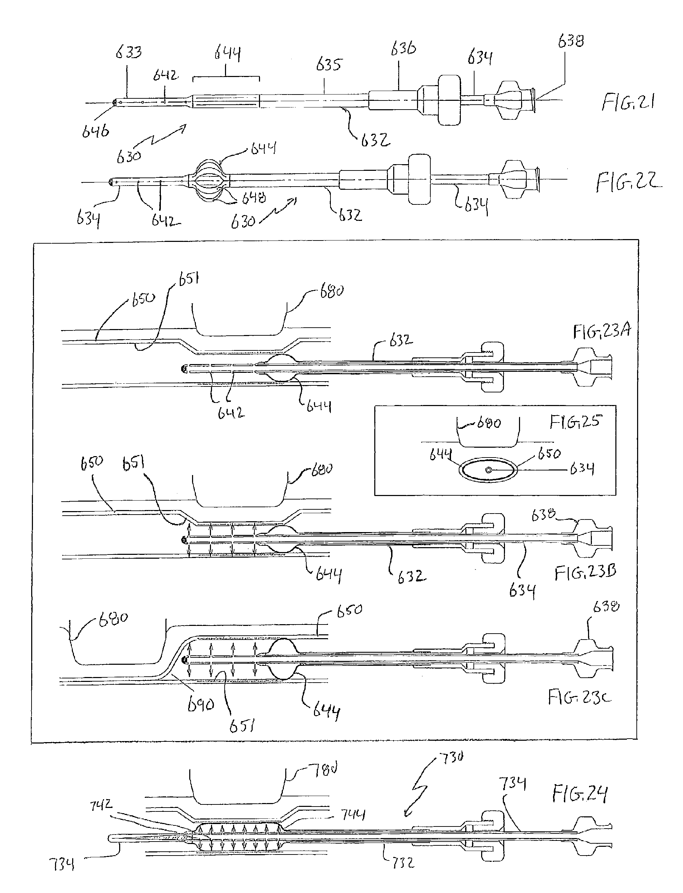

FIGS. 21, 22 and 23A-23C illustrate an embodiment representing another example of the intraluminal device 630 disclosed here. Features in this embodiment which are similar to features described in earlier embodiments are identified by common reference numerals, but preceded by a "6". A detailed description of features in this embodiment that are similar to features in earlier embodiments is not repeated. The configuration of this embodiment of the intraluminal device 630 is the same at the embodiment illustrated in FIGS. 1 and 2. The difference resides in the manner of use or operation of the device. Thus, the following discussion focuses on the differences in operation and use associated with this embodiment.

The intraluminal device 630 is inserted into a vessel (varicose vein) and is advanced to the position in the vein requiring treatment as discussed above. Once the expandable expansion member 644 is positioned at the end (e.g., distal end) of the treatment portion of the vein to be treated, the expandable expansion member 644 is expanded outwardly so that the contact members 648 forming the expanded expansion member 644 contact the inner wall 651 of the vein 650. Following this outward expansion of the expandable expansion member 644, a force or pressure from outside the vein is applied to the vein as illustrated in FIG. 23A. The outside pressure force can be applied by a pressure applier 680 schematically illustrated in FIG. 23A. The pressure applier can be in the form of an ultrasound probe or can be pressure applied by a person, for example using a hand. The pressure applier deforms the vein. The outside pressing force preferably also deforms the expanded expansion member 644.

FIGS. 23A and 23B illustrate the blood vessel 650 and the expanded expansion member 644 being deformed through the application of the outside pressing force. The outside pressure force applied to the vessel by the pressure applier 680 decreases the diameter of the vessel (and the inner and outer diameter of the expanded expansion member 644). In this illustrated example, the blood vessel 650 and the expanded expansion member 644 are deformed to take on an oval or ellipsoidal shape.

The outside pressure force applied by the pressure applier 680 facilitates intimate and strong contact between the expanded expansion member 644 (i.e., the contact members 648 forming the expanded expansion member 644) and the inner wall 651 of the vessel 650. This thus helps ensure an appropriate and desired degree of damage to the vessel by way of the expanded expansion member 644 as the expanded expansion member 644 is axially moved along the longitudinal axis of the vessel. FIG. 23A illustrates the expanded expansion member 644 after axially moving while under the pressure force from the pressure applier. That is, before the application of the outside pressure, the expandable expansion member 644 is positioned left of the position shown in FIG. 23A (generally at left end of the pressure applier 680 shown in FIG. 23A), then the expandable expansion member 644 is expanded outwardly, then the outside pressure force is applied by the pressure applier and the expanded expansion member 644 moves axially to the position shown in FIG. 23A. As illustrated in FIG. 23B, fluid is then introduced into the fluid injector 638, flows along the lumen in the inner tube 634 and is ejected through the through holes 642 in the inner tubular member 634. During this ejection of the fluid, the axial movement of the expanded expansion member 644 is preferably stopped. When the intraluminal device is completely compressed in the vein, the inner surface of the spiral contact members forming the expansion member 644 become flattened, the inner surface of the spiral contact members contacts the inner elongated member so that the spiral members (flattened portion) and the inner elongated member become more rigid or more stiff than in the original shape.

FIG. 25 illustrates the blood vessel 650 and the expanded expansion member 644 being deformed through the application of the outside pressing force. The outside pressure force applied to the vessel by the pressure applier 680 decreases the diameter of the vessel (and the inner and outer diameter of the expanded expansion member 644). In this illustrated example, the blood vessel 650 in front of the expanded expansion member 644 is deformed to take on an oval or ellipsoidal shape. The application of the outside pressure force from the pressure applier 680, the axial movement of the expanded expansion member 644 and the ejection of the fluid into the vessel 650 continues in the manner described above until the entirety of the portion of the vessel (varicose vein) to be treated is treated.

The embodiment of the intraluminal device shown in FIGS. 21, 22 and 23A-23C is configured so that it is possible to damage the inner wall with the expanded expansion member using one hand that axially moves the intraluminal device and to inject fluid into the injection port 638 using the same hand.

The outside pressure force by the pressure applier can cause portions of the inner wall of the blood vessel to not only firmly contact the expanded expansion member (i.e., the contact members 648 forming the expanded expansion member 644) but to also actually enter the spaces between the spiral members. That is, through application of sufficient outside pressure force by the pressure applier 680, portions of the inner wall of the blood vessel will actually enter into the spaces between the circumferentially spaced apart contact members (spiral contact members) to thereby further enhance damage to the blood vessel.

After the expansion member 644 expands and moves from the left end of the outside pressure applier 680 to the right side of the outside pressure applier 680, the fluid is ejected from the through holes 642 in the distal portion of the intraluminal device 630 and then the outside pressure applier 680 is located relatively distal of the distal portion of the intraluminal device 630. The intraluminal device 630 can move toward the proximal end of the vein, while the outside pressure applier keeps the original position. The outside pressure applier 680 can move toward the distal end of the vein, while the intraluminal device keeps the original position. In this state as shown in FIG. 23C, the outside pressure applier 680 is located distal of the distal portion of the intraluminal device 630. The inner wall 651 of the vein 650 is collapsed by the outside pressure applier 680. After the vein is collapsed, the fluid is ejected in the vein from the through holes 642 of the distal portion. The ejected fluid does not flow distally toward the distal side of the vein, but rather flows proximally toward the proximal side or stays where the fluid is ejected from through holes 642 of the distal portion. Collapsing a portion of the vein, the extent of the damages that fluid gives becomes rather extensive. As the fluid used in the steps of FIG. 23B and 23C, adhesive or sclerosant can be used.

FIG. 24 illustrates an embodiment representing another example of the intraluminal device and method disclosed here. In this embodiment, the through holes through which the fluid (sclerosant or adhesive) is ejected are located in the inner tube and are positioned inside the expandable expansion member 744. In this embodiment, the expandable expansion member 744 is expanded as generally illustrated in FIG. 24 and is expanded into contact with the inner wall of the blood vessel to which the exterior force is applied. That is, in this embodiment, the intraluminal device is positioned in the vein and located at the desired place at which treatment is to begin, then the outside pressure force is applied by the pressure applier as described above, the expandable expansion member 744 is outwardly expanded into contact with the inner wall of the vein, fluid is ejected into the vein by way of the through holes in the inner tube, the fluid ejection stops, and the expanded expansion member is axially moved. The applied outside pressure force can cause the inner wall 751 of the blood vessel to contact the through holes 742 of the elongated inner member 634. In this regard, the inner wall of the blood vessel can penetrate the spaces between the contact members of the expanded expansion member 744 when the blood vessel and the expansion member deform.

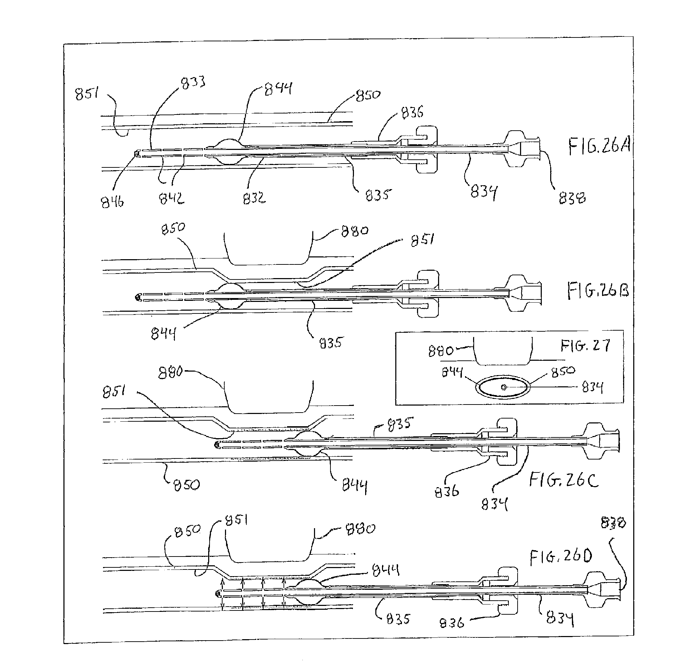

FIGS. 26A-26D illustrate an embodiment representing another example of the intraluminal device disclosed here. Features in this embodiment which are similar to features described in earlier embodiments are identified by common reference numerals, but preceded by an "8". A detailed description of features in this embodiment that are similar to features in earlier embodiments is not repeated. The configuration of this embodiment of the intraluminal device 830 is the same at the embodiment illustrated in FIGS. 1 and 2. The difference resides in the manner of use or operation of the device. Thus, the following discussion focuses on the differences in operation and use associated with this embodiment.

The intraluminal device 830 is inserted into a vessel (varicose vein) and is advanced to the position in the vein requiring treatment as discussed previously. When the expandable expansion member 844 is positioned at the end (e.g., distal end) of the treatment portion of the vein to be treated, the expandable expansion member 844 is expanded outwardly so that the contact members 848 forming the expanded expansion member 844 are outwardly expanded. In this embodiment, the expandable expansion member 844 is expanded outwardly so that the contact members 848 are spaced from the inner wall 851 of the vein 850 as shown in FIG. 26A. Following this outward expansion of the expandable expansion member 844, a force or pressure from outside the vein is applied to the vein as illustrated in FIG. 26B. The outside pressure force can be applied by a pressure applier 880 schematically illustrated in FIG. 26B. The pressure applier can be in the form of an ultrasound probe or can be pressure applied by a person, for example using a hand. The pressure applier deforms the vein and causes the inner wall surface of the vessel (vein) to contact the outer surface of the expanded expansion member 844 (contact members 848). The outside pressing force preferably also deforms the expanded expansion member 844 (contact members 848) as illustrated in FIG. 27.

FIG. 26B illustrates that the blood vessel 850 and the expanded expansion member 844 are deformed through the application of the outside pressing force. The outside pressure force applied to the vessel by the pressure applier 880 decreases the diameter of the vessel (and the inner and outer diameter of the expanded expansion member 844). In this illustrated example, the blood vessel 850 and the expanded expansion member 844 are deformed to take on an oval or ellipsoidal shape. Following the application of the pressure by the pressure applier 880, the expanded expansion member 844 is axially moved along the longitudinal axis of the vessel. This can be seen based on a comparison of FIGS. 26B and 26C.

The outside pressure force applied by the pressure applier 880 facilitates intimate and strong contact between the expanded expansion member 844 (i.e., the contact members 848 forming the expanded expansion member 844) and the inner wall 851 of the vessel 850. An appropriate and desired degree of damage to the vessel by way of the expanded expansion member 844 is thus achieved as the expanded expansion member 844 is axially moved along the longitudinal axis of the vessel.

As illustrated in FIG. 26D, following the axial movement of the expanded expansion member 844, fluid (e.g., a liquid such as a sclerosant or adhesive) is introduced into the fluid injector 838, flows along the lumen in the inner tube 834 and is ejected through the through holes 842 in the inner tubular member 834. During this ejection of the fluid, the axial movement of the expanded expansion member 844 is preferably stopped. When the intraluminal device is completely compressed in the vein, the inner surface of the spiral contact members forming the expansion member 844 become flattened, the inner surface of the spiral contact members contact the inner elongated member so that the spiral members (flattened portion) and the inner elongated member become more stiff or more rigid than in the original shape.

Following the ejection of fluid as shown in FIG. 26D, the operation is repeated, beginning with the operation (step) shown in FIG. 26A or the operation (step) shown in FIG. 26B. When the operation shown in FIG. 26D is followed by FIG. 26B involving applying the outside pressure force to the expanded expandable member 844, the pressure applier 880 shown in FIG. 26D can be moved axially to the right in FIG. 26D so that the left end of the pressure applier 880 overlies the expanded expandable member, or the pressure applier 880 shown in FIG. 26D can be maintained in its illustrated position and a new pressure applier (second pressure applier) is positioned axially adjacent to the pressure applier shown in FIG. 26D so that the left end of the new (second) pressure applier overlies the expanded expandable member.

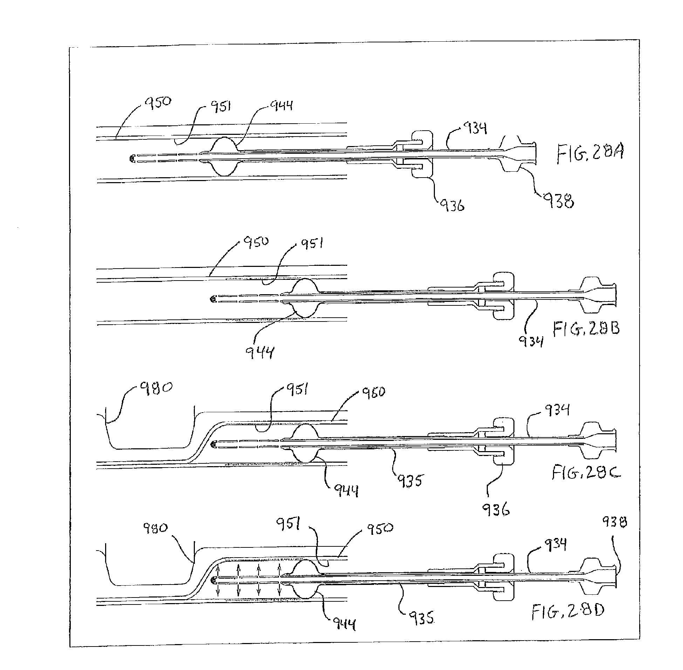

FIGS. 28A-28D illustrate an embodiment representing another example of the intraluminal device disclosed here. Features in this embodiment which are similar to features described in earlier embodiments are identified by common reference numerals, but preceded by a "9". A detailed description of features in this embodiment that are similar to features in earlier embodiments is not repeated. The configuration of this embodiment of the intraluminal device 930 is the same at the embodiment illustrated in FIGS. 1 and 2. The difference resides in the manner of use or operation of the device. Thus, the following discussion focuses on the differences in operation and use associated with this embodiment.

The intraluminal device 930 is inserted into a vessel (varicose vein) and is axially moved (advanced) to the position in the vein requiring treatment as discussed previously. When the expandable expansion member 944 is positioned at the end (e.g., distal end) of the treatment portion of the vein to be treated, the expandable expansion member 944 is expanded outwardly so that the contact members 948 forming the expanded expansion member 944 are outwardly expanded. In this embodiment, the expandable expansion member 944 is expanded outwardly so that the contact members 848 directly contact the inner wall 951 of the vein 950 as shown in FIG. 28A. Following this outward expansion of the expandable expansion member 944 and contact of the contact members with the inner wall of the vein, the expanded expansion member 944 is axially moved along the longitudinal axis of the vessel. This can be seen based on a comparison of FIGS. 28A and 28B.

Next, a force or pressure from outside the vein is applied to the vein as illustrated in FIG. 28C. The outside pressure force can be applied by a pressure applier 980 similar to the pressure applier described above. The outside pressure is applied to the vein at a position upstream of the position of the expanded expansion member 944, considered with reference to the direction of axial movement of the expanded expansion member 944. More specifically, the outside pressure is applied to the vein at a position upstream of the position of the distal-most end of the intraluminal device 930. The pressure applier deforms the vein and causes the inner wall surface of the vessel (vein) to collapse and close. In this state as shown in FIG. 28C, the outside pressure applier 980 is located distal of the distal portion of the intraluminal device. The inner wall 951 of the vein 950 is collapsed by the outside pressure applier 980. After the vein is collapsed, the fluid is ejected in the vein from the through holes of the distal portion. By virtue of the outside pressure applier 980 which collapses the vein, the ejected fluid does not flow distally toward the distal side of the vein, but instead flows proximally toward the proximal side or stays where the fluid is ejected from through holes of the distal portion. Collapsing a portion of the vein, the extent of the damages that fluid gives becomes rather extensive.

Following application of the outside pressure by the pressure applier 988, fluid (e.g., a liquid such as a sclerosant or adhesive) is introduced into the fluid injector, flows along the lumen in the inner tube 934 and is ejected through the through holes 942 in the inner tubular member 934 as shown in FIG. 28D. During this ejection of the fluid, the axial movement of the expanded expansion member 944 is preferably stopped.

Following the ejection of fluid shown in FIG. 28D, the operation is repeated, beginning with the operation (step) shown in FIG. 28A or the operation (step) shown in FIG. 28B.

FIGS. 29A-29C illustrate various configurations for the expandable expansion member. In particular, FIGS. 29A-29C show different configurations for the expandable contact members forming the expandable expansion member. FIG. 29A shows an expandable spiral expansion member with contact members like those forming the expandable spiral expansion illustrated in FIG. 4, FIG. 29B illustrates an expandable expansion member formed from a step-wise spiral arrangement of contact members, and FIG. 29C illustrates an expandable expansion member formed from a braided arrangement of contact members.

The detailed description above describes features and aspects of examples of embodiments of a vein treatment method and assembly/intraluminal device. The present invention is not limited, however, to the precise embodiments and variations described. Various changes, modifications and equivalents could be effected by one skilled in the art without departing from the spirit and scope of the invention as defined in the appended claims. It is expressly intended that all such changes, modifications and equivalents which fall within the scope of the claims are embraced by the claims. For example, the treatment method of treating a vein (varicose vein) can be performed by more than two people (a plurality of people).

## Claims

1. A device configured to be positioned in a vein to treat the vein, the device comprising:
an outer sheath possessing a proximal end portion and a distal end portion;
an elongated inner member positioned inside the elongated outer sheath, the elongated inner member possessing a distal end portion;
an expandable spiral expansion member possessing a proximal end portion and a distal end portion, the spiral expansion member being positioned between the distal end of the elongated outer sheath and the distal end portion of the elongated inner member, the elongated inner member being fixed to the distal end portion of the expandable spiral expansion member, the distal end portion of the elongated outer sheath being fixed to the expandable spiral expansion member;
the elongated inner member being axially movable relative to the elongated outer sheath when the device is positioned in the vein so that axial movement of the elongated inner member in a proximal direction relative to the elongated outer sheath axially moves the distal end of the expandable spiral expansion member toward and relative to the proximal end of the expandable spiral expansion member such that the expandable spiral expansion member outwardly expands to an expanded state so that the spiral expansion member becomes an expandable spiral expansion member comprising a plurality of spirally extending contact members in contact with the inner wall of the vein;
the expandable spiral expansion member being axially movable along, and rotatable relative to, the vein while the spirally extending contact members are in contact with the inner wall of the vein to damage the inner wall of the vein.

2. The device according to Claim 1, wherein the elongated inner member possesses a distal end portion located distal of the expandable spiral expansion member, the elongated inner member including a lumen, the distal end portion of the elongated inner member including a plurality of through holes communicating with the lumen in the elongated inner member.

3. The device according to Claim 1, wherein the expandable spiral expansion member is adapted to be rotated automatically as the expandable spiral expansion member is moved axially.

4. The device according to Claim 1, wherein the intraluminal device possesses a longitudinal axis, and the expandable spiral expansion member is adapted to be rotated by rotating the expandable spiral expansion member about the longitudinal axis of the intraluminal device.

5. The device according to Claim 1, wherein the expandable spiral expansion member is adapted to be rotated by rotating the expandable spiral expansion member along a spiral direction.

6. The device according to Claim 1, wherein the elongated inner member possesses a distal-most end spaced a first distance from a distal-most end of the expandable spiral expansion member.

7. The device according to Claim 1, further comprising a hub connected to the proximal end portion of the outer sheath, the elongated outer sheath including a lumen and a plurality of through holes in the outer sheath, the plurality of holes being arranged along an axially extending portion of the outer sheath located between the spiral expansion member and the hub, the through holes communicating with the lumen in the elongated outer sheath.

8. The device according to Claim 1, further comprising an elongated inner tube positioned inside the elongated outer sheath and the expandable expansion member, the elongated inner tube possessing a distal end portion located distal of the expandable expansion member, the elongated inner tube including a lumen, the distal end portion of the elongated inner tube including a plurality of through holes communicating with the lumen in the elongated inner tube.

9. The device according to Claim 1, wherein the expandable expansion member comprises a plurality of circumferentially spaced apart and spirally extending contact members, and the device is adapted for the rotation of the expandable expansion member occurring automatically as the expandable expansion member is moved axially by virtue of the spirally extending contact members contacting the inner wall of the vein.

10. The device according to Claim 1, further comprising an elongated inner tube positioned inside the elongated outer sheath and the expansion member, the elongated inner tube possessing a distal-most end spaced a first distance from a distal-most end of the expandable expansion member.

11. The device according to Claim 1, further comprising a hub connected to the proximal end portion of the elongated outer sheath, the elongated outer sheath including a lumen and a plurality of through holes communicating with the lumen in the elongated outer sheath, the plurality of through holes in the elongated outer sheath being positioned between the expansion member and the hub.
